# EUROPEAN PATENT APPLICATION

(11) **EP 1 688 429 A1**
(43) Date of publication of application: **09.08.2006**
(21) Application number: 05002356.3
(22) Date of filing: 04.02.2005
(51) Int. Cl.: C07K 14/415, A61P 35/00, A61P 37/02

(54) **Riproximin, a novel type II ribosome-inactivating protein and uses thereof**

(71) Applicant: Deutsches Krebsforschungszentrum Stiftung des öffentlichen Rechts, 69120 Heidelberg (DE)
(72) Inventor: Voss, Cristina, 69245 Bammental (DE); Berger, Martin, 69126 Heidelberg (DE); Schumacher, Carlos, 22391 Hamburg (DE)
(74) Representative: Schüssler, Andrea

(57) **Abstract**

Described is a novel type II ribosome-inactivating protein, riproximin, as well as nucleic acid molecules encoding said protein. Furthermore, therapeutic uses of riproximin are described.

## Description

The present invention relates to a novel type II ribosome-inactivating protein, riproximin, as well as nucleic acid molecules encoding said protein. Furthermore, the present invention relates to therapeutic uses of riproximin.

Plant ribosome-inactivating proteins (RIPs) are a group of plant proteins with RNA-glycosidase activity which act on eukaryotic and prokariotic ribosomes. While some RIPs (e.g. the type II RIPs ricin, abrin, volkesin, viscumin) are highly toxic to humans and higher animals, other members of this class show only a low or no toxicity (e.g. the type II RIPs ebulin, nigrin, cinnamomin as well as all known type I and type III RIPs). The molecular mechanism of action of RNA N-glycosidase is to remove a specific adenine from a highly conserved loop ("sarcin/ricin domain") in the largest ribosomal RNA that is responsible for the interaction of both eukaryotic and prokaryotic elongation factors with the ribosome, thereby inhibiting protein synthesis. RIPs are classified into three types based on their primary structure. Type I RIP consists of a single, intact polypeptide of about 30kDa which, in some cases, is processed proteolytically into two shorter polypeptides held together by non-covalent interactions. Type II RIP is composed of an N-terminal domain comparable with type I RIP linked by a disulphide bridge to a C-terminal domain that has carbohydrate-binding activity. Type III RIP consists of a type I RIP-like N-terminal domain and a C-terminal domain of unknown function.

Many studies have been performed on the applications of RIPs in drug development, immuno-modulation and crop-plant biotechnology due to their toxicity to viruses, tumour cells, insects and plant fungal pathogens. RIPs have also been used as a powerful probe to study the structure of ribosomes. However, the physiological function that RIPs play in the plant cell is unclear. Previous studies revealed that many RIPs are involved in defence mechanisms in plant cells and terminate protein synthesis under appropriate physiological conditions and thus are involved in metabolic regulation. RIPs occur in various plant tissues but are most prominent in storage organs like cotyledon, endosperm, bark, tubers, storage roots, bulbs and rhizomes. Apart from a few exceptions, RIPs occur as mixtures of more or less closely related isoforms; see van Damme et al., Crit.Rev.Plant Sci. 20 (2001), 395-465.

One member of the type II RIP family is viscumin, a toxic lectin from mistletoe. It was demonstrated that viscumin is remarkably similar in structure and mechanism of action to the plant toxins abrin, ricin, and modeccin and that one of the constituent peptide chains of viscumin, the A chain, inhibits cell-free protein synthesis by inactivating catalytically the large ribosomal subunit. Moreover, viscumin seems to be a promising drug candidate for cancer therapy and phase I/II clinical trials are currently carried out. Thus, there is a need for the isolation and characterization of further members of the type II RIP family which might be useful for cancer therapy.

Thus, the technical problem underlying the present invention is to provide alternative type II RIPs which might be useful for therapy of tumors, modulation of the immune system, antiviral treatment, protection against insects and plant fungal pathogens.

The solution to said technical problem is achieved by providing the embodiments characterized in the claims. During the experiments resulting in the present invention a protein from *Ximenia americana* could be isolated and characterized. This protein, which was named riproximin, is a member of the type II RIPs and shows antineoplastic activities, thus is useful, for example, for therapy of tumours, modulation of the immune system, antiviral treatment, protection against insects and plant fungal pathogens.

### BRIEF DESCRIPTION OF THE DRAWINGS

### Figure 1: In vitro comparison of the anti-neoplastic activity of an aqueous extract from Ximenia Americana containing riproximin in 16 human and one rodent cell lines

Extract concentrations describing IC₅₀ values are given in µg crude extract/ml, i.e. the amount (µg) of dry *Ximenia americana* powder used for the preparation of the respective dilution. The average IC₅₀ value of all cell lines was 49 µg crude extract/ml medium.

### Figure 2: In vivo antitumor activity of an aqueous extract containing riproximin, following tannin pre-extraction from Ximenia Americana

The effect of the aqueous extract containing riproximin, following tannin pre-extraction, was analyzed by the CC531 colon carcinoma liver metastasis model (Wittmer et al., Clin.Exp.Met. 17 (1999), 369-376). The maximum effect of the antimetabolite Alimta is given for comparison. Extract concentrations are given in mg crude extract/kg body weight, i.e. the amount (mg) of dry *Ximenia americana* powder used for the preparation of the respective dilution.

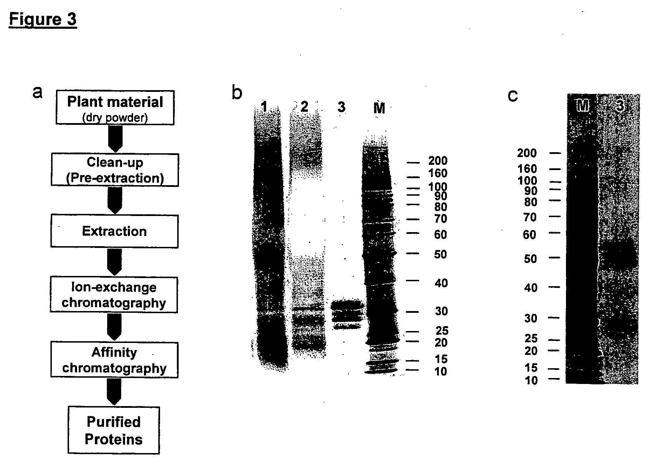

### Figure 3: Purification of the anti-neoplastic protein, riproximin

**(a)** Purification flow chart;
(b) SDS-PAGE (under reducing conditions) of crude extract after tannin extraction (1), total protein fraction after ion-exchange chromatography (2), and affinity-chromatography purified proteins (3); M, molecular weight markers (MW in kDa).
**(c)** SDS-PAGE (under non-reducing conditions) of affinity chromatography purified proteins (3); M, molecular weight markers (MW in kDa).

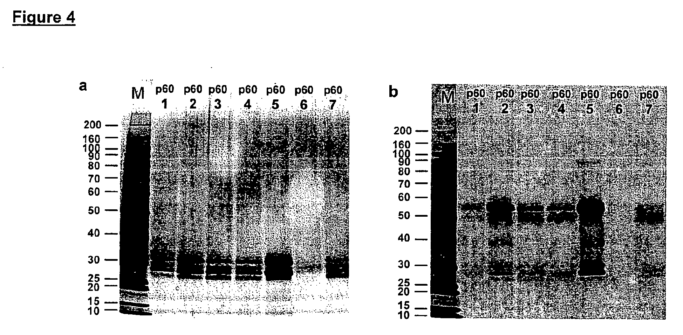

### Figure 4: Various affinity-purified protein preparations

Lanes 1-7: 7 different batches of riproximin were analyzed by reducing SDS-PAGE **(a)** or non-reducing SDS-PAGE **(b).** M = molecular weight markers (weight in kDa). Protein concentrations and IC₅₀ values are given in ng dry protein/ml. The amount (ng) of dry protein in the affinity purified fraction was determined by lyophilisation.

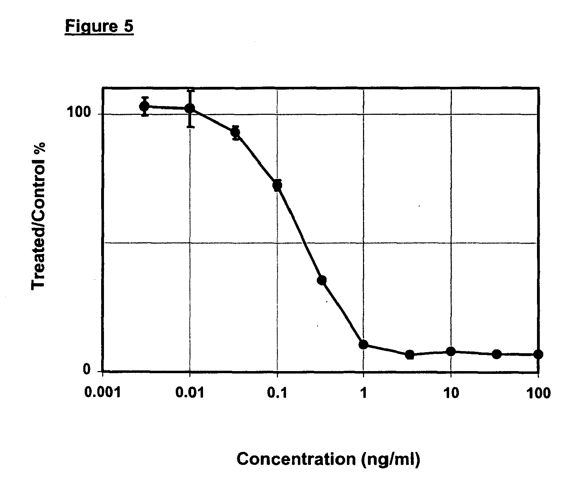

### Figure 5: Cytotoxic activity of an affinity-purified riproximin preparation in MCF7 breast cancer cells

The viable cell count was determined by the MTT-assay (Konstantinov et al., Int.J.Cancer 77 (1998), 778-86). The effect of increasing concentrations (ng/ml medium) is given in percent of untreated control.

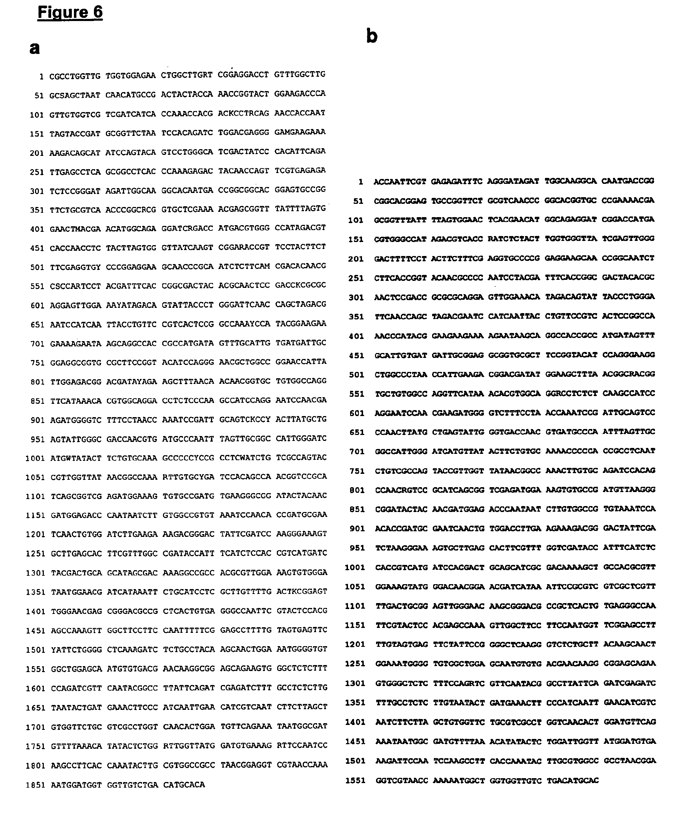

### Figure 6: Nucleotide sequence of the riproximin encoding gene

**(a)** complete cDNA sequence derived from *Ximenia Americana* of US-origin (SEQ ID NO:1).
(b) incomplete coding genomic DNA sequence derived from *Ximenia Americana of Tanzanian origin* (SEQ ID NO:2). About 250 bp are missing at the 5'end.

The US- and Tanzanian riproximin DNA sequences share 95 - 97% identity, depending on the respective allelic state.

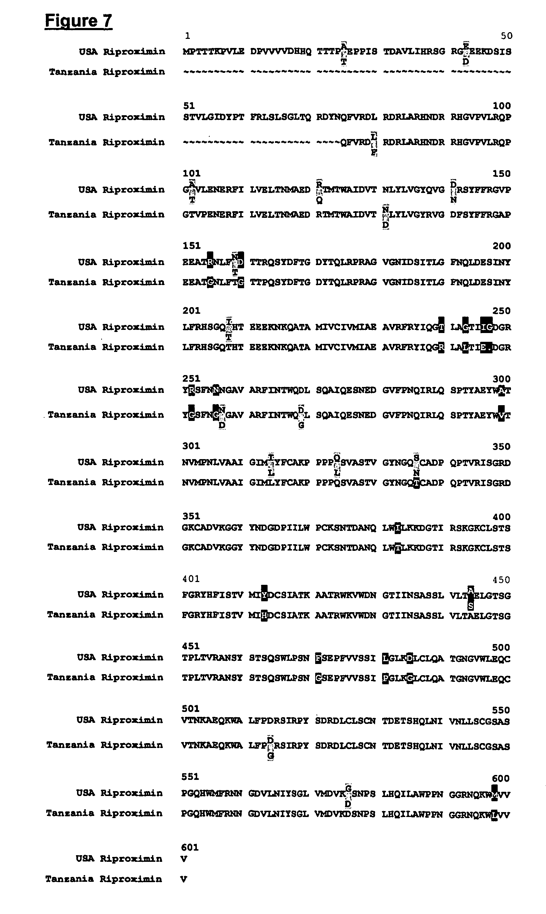

### Figure 7: Derived amino acid sequence of riproximin

The complete amino acid sequences of US-riproximin (SEQ ID NO:3) as well as an incomplete C-terminal sequence of the Tanzanian riproximin (SEQ ID NO:4) were aligned. Allelic amino acid variations are indicated by giving the respective abbreviations in super- and subscript, and light grey highlighted. Amino acids differing between the US and Tanzanian proteins are given in white and highlighted in black.

Allelic riproximin amino acid sequences show 97.9% similarity and 97.8% identity. Riproximin amino acid sequences of US- and Tanzanian origin share 94.9 - 96.2% similarity and 94.3-95.8% identity, depending on the respective allelic state (percent identity=percent of identical aminoacids within the two sequences; percent similarity=percent identical and similar aminoacids (aminoacids with the same function) between the two sequences).

Thus, the present invention relates to a nucleic acid molecule encoding the plant type II ribosome-inactivating protein (RIP) riproximin exhibiting anti-neoplastic activity or a polypeptide exhibiting a biological property of riproximin, which is:
(a) a nucleic acid molecule encoding a polypeptide that comprises the amino acid sequence as depicted in Figure 7 (SEQ ID NOs:3 and 4);
(b) a nucleic acid molecule comprising the nucleotide sequence as depicted in Figure 6(a) (SEQ ID NO:1) or (b)(SEQ ID NO:2);
(c) a nucleic acid molecule encoding a polypeptide the sequence of which shows at least 70%, preferably at least 80%, 85%, 90%, 95% or 98%, in the most preferred embodiment, 98% identity to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code; or
(e) a nucleic acid molecule, which represents a fragment or a variant of a nucleic acid molecule of (a) to (d).

As used herein, a polypeptide exhibiting a biological property of riproximin is understood to be a polypeptide having at least one of the biological activities of a type II RIP. These activities include cytotoxic activity *in vitro* and in vivo, immunomodulatory activity *in vivo,* inhibition of protein synthesis, specific hydrolysis of the A⁴³²⁴ in the ricin-sarcin loop of eukaryotic 28S rRNA, polynucleotide N-glycosylase activity on DNA or RNA, antiviral, antifungal and insecticide activity.

In a first embodiment, the invention provides a nucleic acid molecule encoding an riproximin polypeptide that comprises the amino acid sequence as depicted in Figure 7 (SEQ ID NOs:3 or 4) .

The present invention also provides a nucleic acid molecule encoding an riproximin polypeptide comprising the nucleotide sequence as depicted for DNA in Figure 6(a) (SEQ ID NO:1) or (b) (SEQ ID NO:2).

The nucleic acid molecules of the invention can be both DNA and RNA molecules. Suitable DNA molecules are, for example, genomic or cDNA molecules. It is understood that all nucleic acid molecules encoding all or a portion of riproximin are also included, as long as they encode a polypeptide with biological activity. The nucleic acid molecules of the invention can be isolated from natural sources or can be synthesized according to known methods.

The present invention also provides nucleic acid molecules encoding a polypeptide the amino acid sequence of which shows at least 70%, preferably at least 80%, 85%, 90%, 95% or 98%, in the most preferred embodiment, 98% identity to the amino acid sequence of the polypeptide of Figure 7 (SEQ ID NOs:3 or 4). Such amino acid sequences are characterized by deletion, substitution and/or insertion of amino acid residue(s) compared to the amino acid sequences shown in Figure 7 (SEQ ID NOs:3 or 4) or are the result of recombination. They can be naturally occurring variations, for example sequences from other organisms, or mutations that can either occur naturally or that have been introduced by specific mutagenesis. They can also be isolated, e.g., from genomic or cDNA libraries that were produced from plant cells or tissues. In order to identify and isolate such nucleic acid molecules the molecules of the invention or parts of these molecules or the reverse complements of these molecules can be used, for example by means of hybridization. As a hybridization probe nucleic acid molecules can be used, for example, that have exactly or basically the nucleotide sequence as depicted in Figure 6 (SEQ ID NOs:1 or 2), respectively, or parts of these sequences. The fragments used as hybridization probe can be synthetic fragments that were produced by means of conventional synthetic methods and the sequence of which basically corresponds to the sequence of a nucleic acid molecule of the invention.

The nucleic acid molecules of the present invention also include molecules which differ from the nucleic acid molecules with sequences shown in Figure 6(a) (SEQ ID NO:1) or (b) (SEQ ID NO:2) due to the degeneracy of the genetic code.

In a further embodiment, the present invention provides nucleic acid molecules which comprise fragments or variants of the nucleic acid molecules described above encoding a polypeptide of the invention which still show a biological activity. "Fragments" are understood to be stretches of DNA and are parts of the nucleic acid molecules that are long enough to encode one of the described polypeptides. These fragments also comprise nucleic acid molecules specifically hybridizing to (transcripts of) the nucleic acid molecules of the invention. These nucleic acid molecules can be used, for example, as (gene) probes or primers, e.g., for isolating homologous genes from related plants and, preferably, are oligonucleotides having a length of at least 10, in particular of at least 15 and particularly preferred of at least 50 nucleotides. The nucleic acid molecules and oligonucleotides of the invention can also be used, for example, as primers for a PCR reaction.

The variants can be either naturally occurring variants or synthetically produced variants or variants produced by recombinant DNA processes. Naturally occurring variants include variants between subspecies of *Ximenia Americana* (e.g., *X.a.* of African or American origin), as well as isoenzymes occurring in the same species, with amino acid sequences showing more than 70% identity to the amino acid sequence depicted in Figure 7 (SEQ ID NOs:3 or 4).

Generally, by means of conventional molecular biological processes it is possible (see, e.g., Sambrook et al., supra) to introduce different mutations into the nucleic acid molecules of the invention. As a result, riproximin polypeptides or riproximin related polypeptides with possibly modified biological properties are synthesized. One possibility is the production of deletion mutants in which nucleic acid molecules are produced by continuous deletions from the 5'- or 3'-terminus of the coding DNA sequence and that lead to the synthesis of polypeptides that are shortened accordingly. Another possibility is the introduction of single-point mutation at positions where a modification of the amino acid sequence influences, e.g., the proteolytic properties. By this method muteins can be produced, for example, that possess a modified Kₘ-value or that are no longer subject to the regulation mechanisms that normally exist in the cell, e.g. with regard to allosteric regulation or covalent modification.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptides encoded by the various variants of the nucleic acid molecules of the invention show some of the common characteristics, such as anti-neoplastic activity, cytotoxic activity *in vitro* and in vivo, immunomodulatory activity in vivo, inhibition of protein synthesis, specific hydrolysis of the A⁴³²⁴ in the ricin-sarcin loop of eukaryotic 28S rRNA, polynucleotide N-glycosylase activity on DNA or RNA, antiviral, antifungal and insecticide activity, immunological reactivity or conformation, lectin activity or physical properties like the solubility.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors or the invention. A host cell is understood to be an organism that is capable to take up *in vitro* recombinant DNA and, if the case may be, to synthesize the polypeptides encoded by the nucleic acid molecules of the invention. Preferably, these cells are prokaryotic or eukaryotic cells, for example mammalian cells, bacterial cells, insect cells, plant cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a polypeptide exhibiting a biological property of riproximin and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an anti-riproximin antibody, or, e.g., can be substantially purified by the one-step method described in Smith and Johnson, Gene 67; 31-40 (1988). These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. The polypeptides are preferably in a substantially purified form. Furthermore, the polypeptides may be further processed, e.g by glycosylation. This glycosylation which may occur within or outside of the production cell may have an influence on the activity and behavior of the polypeptide.

Furthermore, the present invention provides conjugates comprising a protein of the present invention as partner. Such conjugates, preferably immunoconjugates, can be prepared according to methods well known to the person skilled in the art, e.g., by recombinant DNA technology and/or conventional chemical synthesis. Immunoconjugates are, e.g. suitable for reducing proliferation of neoplastic cells. For example, the binding partner of riproximin, i.e. antibody or part thereof as described below is chosen so as to be specificially reactive with one or more neoplastic cell specific internalizing antigens present on the surface of the neoplastic cell. The conjugate is then placed in contact with the neoplastic cells and allowed to bind the neoplastic cell specific internalizing antigen. Once bound, the riproximin or related protein will be internalized through the endocytic pathway. Thus, such conjugates are useful for a variety of therapies, e.g., for treatment of tumours or immunomodulation. A further example of a conjugate of the present invention is a riproximin-ferritin conjugate. The generation of ferritin conjugates is, e.g. described by Schultz et al., Biochemistry 20(12) (1981), 3412-8.

The present invention also relates to an antibody that binds specifically to a riproximin or a related polypeptide as defined above. The term "antibody", preferably, relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

For certain purposes, the antibody of the present invention can be detectably labeled, for example, with a radioisotope, a bioluminescent compound, a chemiluminescent compound, a fluorescent compound, a metal chelate, or an enzyme.

The present invention also relates to a pharmaceutical composition comprising a nucleic acid molecule, polypeptide, conjugate or recombinant vector according to the present invention and a pharmaceutically acceptable excipient, diluent or carrier.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by oral, intravenous, intraperitoneal, subcutaneous, intramuscular, topical, intradermal, intrarterial, intratumoral or extracorporal administration. The route of administration, of course, depends on the nature of the disease, e.g., tumor, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease (e.g. tumor), general health and other drugs being administered concurrently.

The delivery of the nucleic acid molecules of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, e.g., a tumor to be treated, the nucleic acid molecules of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to the use of the above compounds of the invention (nucleic acid molecules, proteins, conjugates, antibodies, vectors etc.) for the preparation of a pharmaceutical composition for treatment of a tumor or for immunomodulation. For such uses, plant extracts containing a protein or nucleic acid of the present invention are also suitable. Such extracts can be prepared according to the methods described in the examples. Preferably, the crude plant extract is at least partially purified, e.g., by aqueous extraction from *Ximenia americana* plant material. The plant extract can be further purified, e.g., by pre-extraction of the plant material, preferably with 70% acetone in water. Additional preferred purification methods suitable for removing protein contaminants include ion exchange and/or affinity chromatography.

The following example illustrates the invention.

### Example

### Characterization of the crude extract obtained from Ximenia americana

### (A) Preparation of crude extract from Ximenia americana

Dry plant material from *Ximenia Americana* was powdered and an aqueous extract was prepared by suspending the powder in water. After a brief centrifugation, the supernatant was collected. For in vitro experiments the crude extract was diluted in culture medium and sterilized by filtration.

### (B) Biological effects

### (1) Cell cultures

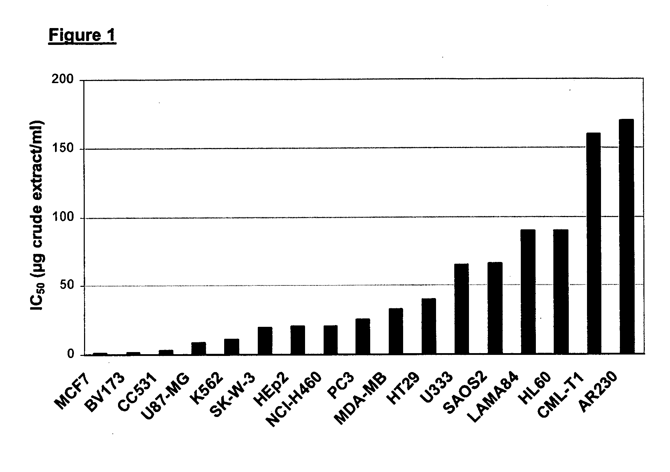

The effects of the aqueous extract on various tumor cell lines (16 human and 1 rodent cell lines) were investigated. The strongest growth inhibition was found for MCF7 breast cancer cells, BV173 chronic myeloid (CM) leukemia, astrocytoma-glioblastoma U87-Mg cells as well as the rat colon carcinoma cell line CC531. The results are summarized in Table 1 and Figure 1. Extract concentrations and IC₅₀ values are given in µg crude extract/ml, i.e. the amount (µg) of dry *Ximenia americana* powder used for the preparation of the respective dilution.

### (2) Animals

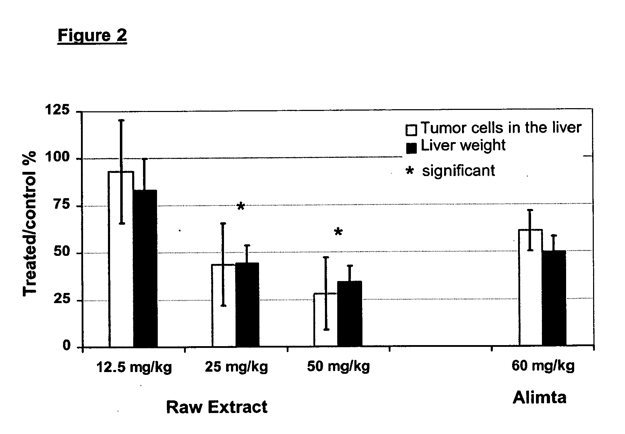

The effect of the aqueous extract *in vivo* was determined by use of the liver metastasis model of the isogenic colon carcinoma cell line CC531. Various doses of the aqueous extract after tannin pre-extraction were applied i.v. for five consecutive days (with day 1 being the day of implantation of tumor cells). The results are shown in Figure 2. For comparison, the maximum effect of the cytostatic compound Alimta in the same animal model is shown (* indicates a significant effect. The multiple nonparametric Kruskal-Wallis test (ADAM, DKFZ Heidelberg) was used to compare tumor cell numbers and liver weights of treated animals with controls; p-values<0,05 were considered significant.).

### (C) Physicochemical characterization of the extract/active agent

### (1) Solubility

Water soluble; PBS: strong growth inhibiting effect *in vitro* (IC₅₀ = 1.8 µg crude extract/ml on MCF7 mamma carcinoma cells); only partially soluble in methanol: the growth inhibiting effect *in vitro* is lower (by a factor of 300; IC₅₀ = 600 µg crude extract/ml on MCF7 mamma carcinoma cells); insoluble in ethanol, acetone, chloroform; can be well extracted with water/methanol mixtures (methanol < 30%; IC50 < 2µg crude extract/ml); can be poorly extracted with water/methanol mixtures (methanol > 70%; IC₅₀ > 333 µg crude extract/ml on MCF7 mamma carcinoma cells); the active compound can be completely extracted after pre-extraction of the powder with ethanol (IC₅₀ = 2 µg crude extract/ml on MCF7 mamma carcinoma cells); pre-extraction with methanol renders a second extraction with water less efficient (IC₅₀ = 14 µg crude extract/ml on MCF7 mamma carcinoma cells). A pre-extraction with 70% acetone in water renders the second extraction with water more efficient (IC₅₀ = 0,7 µg crude extract/ml on MCF7 cells).

### (2) Influence of pH on solubility/biological activity

Extraction with 10 mM buffer/acid/base-solution (pH 2 to 13; short-term exposure of one hour or less) did not show any negative effect. Diluted acids (10 mM HC1, 2 hours at RT) partially destroy the biological activity. Concentrated acids completely destroy the biological activity. Diluted bases (10 mM NaOH, 2 hours at RT) do not show any effect on biological activity, concentrated bases completely destroy the biological activity. Extract concentrations and IC₅₀ values are given in µg crude extract/ml, i.e. the amount (µg) of dry *Ximenia americana* powder used for the preparation of the respective dilution.

### (3) Precipitation with organic solvents

By addition of ethanol, methanol, acetone or acetonitril complete precipitation of the active compound from a aqeous extract can be achieved. The pellet can be completely resolved (water or buffer) without any loss of biological activity.

### (4) Estimation of molecular weight by ultracentrifugation

The active compound completely passes a ultrafiltration membrane having a cut-off value of 100 kDa, partially passes a membrane having a cut-off value of 50 kDa and does not pass a membrane having a cut-off value of 10 kDa.

### (5) Test on tannins (polyphenoles)

The methanol extract contains high concentrations of polyphenoles. By use of C18 reverse phase HPLC/MS various catechines, gallocatechines and gallic acid were identified as components of the extract. A method for extracting tannins from the crude material was established. However, the tannin containing fraction did not show any effect in vitro. In the aqueous extract which had been prepared from the pre-extracted tannin-free crude material polyphenoles could not be identified by HPLC. *In vitro,* the effect of this extract was stronger compared to the effect of the extract which had been prepared from the tannin containing initial sample.

### (6) Results of digestion with trypsin, proteinase K, DNase and RNase

There was no loss of activity after treatment of the aqueous extract prepared from the initial sample for 24h at 37° with trypsin, proteinase K, RNase or DNase. However, the effect of the tannin-free extract was completely abolished after proteinase K treatment (but not after treatment with trypsin).

### (D) Stability of the biological activity

### (1) Stability of the Ximenia Americana dry powder and reproducibility

After dry storage of the powder at 4°C the biological activity was stable for more than three years. Samples of the powder that were assayed after 1, 2 or 3 years showed a practically constant effect *in vitro.*

### (2) Stability of the aqueous extract

Loss of activity up to 20% (after one day), 50% (after one weak) and 80% (after one month) when stored at 4°C was observed. If stored at -20°C for 1 year there was no loss of activity.

### (3) Temperature sensitivity of the aqueous extract

After storage at RT for 2 to 3 h no loss of activity was observed. Slight loss of activity was observed after 10 to 30 min at 50°. After incubation at 90°C for 10 min, the activity was completely destroyed.

### (4) Lyophilization

After lyophilization no loss of activity was observed. 97% of the lyophilized material can be re-disolved in water or PBS. The lyophylized material is stable over a period of one year if stored in the dry at 4°C or in solution at -20°C.

Conclusion: The stability properties of the extract show that the active compound is a protein.

### (5) Binding- and purification characteristics

C18 resins: aqueous extract, pH 7.0: no binding of the active compound. The methanol eluate of the column contains polyphenoles and catechines.
Cation exchange resins: No binding of the active compound at pH 7.0 in 20 mM Tris-HCl. No measurable purification of the extract.
Anion exchange resins: At pH 7.0 in 20 mM Tris-HCl there is binding of the active compound to weak and strong anion exchange media. Active fractions can be eluted with 20 mM Tris-HCl, pH 7.0, with addition of 200 - 400 mM NaCl. Two different fractions can be partially separated. Components of the extract which strongly bind to the resin can only be eluted by NaOH.
Affinity binding: The active compound binds to partially hydrolyzed Sepharose, i.e., shows an affinity to galactose residues. After washing, an active fraction can be eluted from the material by use of a buffer containing 0.1 M galactose.

### (E) Purification of the active compound

The results are shown in Figure 3. The flow chart of the purification of the active compound is given in Figure 3a. The achieved purification grade was monitored by SDS-gel electrophoresis (Figure 3b). After a pre-extraction with an admixture of 70% acetone in water, the plant powder was pelleted by centrifugation and air-dried. A second extraction was performed by suspending the dried pre-extracted powder in water. The undissolved material was again spun down and discarded. The supernatant was adjusted to pH=7.0 by addition of 1M Tris-HCl buffer and applied on a DEAE ion-exchange column. After elution of the unbound components, the active fraction was eluted with 20 mM Tris-HCl buffer (pH=7.0) containing 500 mM NaCl. The support for the affinity chromatography was prepared by hydrolysing Sepharose 2B in 1 M HCl for 3h at 50°C. The ion-exchange-purified fraction was applied to a chromatography column containing the hydrolysed Sepharose. Unbound components were washed out with 20 mM Tris-HCl buffer (pH=7.0) containing 500 mM NaCl and the active fraction was eluted with 20 mM Tris-HCl buffer (pH=7.0) containing 500 mM NaCl and 100 mM galactose. Two proteins could be identified which could be partially separated from each other (Figure 3c). Both proteins have a MW of about 60 kDa and each are composed of two subunits with a MW of 26 - 32. By comparing the biological effects of various fractions having different protein compositions it was found that both proteins exhibit an anti-neoplastic effect. One of the proteins was further characterized and the corresponding gene was sequenced.

### (F) Characterization of the active compound

### (1) Analysis by mass spectroscopy

One of the purified proteins was subjected to analysis by MS-MS mass spectroscopy. The amino acid sequence of some of the MS-identified peptides was determined. It was found that three of these peptides showed homologies to a class of already known proteins, the so-called type II ribosomal inhibiting proteins (RIPs).

### (2) Sequencing

On the basis of the amino acid sequences of the peptides, degenerated primers were developed which allowed to clone a 410 bp fragment. From this sequence information, the complete cDNA was sequenced, using RNA from *Ximenia Americana* of US-origin. The sequence revealed the existence of two alleles, which share more than 95% sequence identity (Figure 7) (SEQ ID NOs:3 and 4). Similarly, the incomplete coding genomic DNA sequence for riproximin was obtained from *Ximenia Americana* from Tanzanian origin and showed over 94% identity to the DNA and amino acid sequence of US-origin, respectively (Figure 6(a)(SEQ ID NO:1)+(b)(SEQ ID NO:2). The translated amino acid sequence identifies the new protein, which was named riproximin, as a member of the type II RIP family. Table 2 gives a comparison of the amino acid sequence of riproximin with that of known type II RIPs. The percent similarity and identity between the amino acid sequences of riproximin and the other type II RIPs did not exceed 58% and 50%, respectively.

### (3) Biological activity

The proteins in the affinity-purified active fraction showed very high levels of cytotoxicity in MCF7 breast cancer cells *in vitro,* with an IC50 of 0.2 ng/ml (Figure 5). Protein concentrations and IC₅₀ values are given in ng dry protein/ml.

The amount (ng) of dry protein in the affinity purified fraction was determined by lyophilisation.

**Table 1: Antiproliferative activity of an aqueous extract from Ximenia americana containing riproximin in 16 human and one rodent cell lines.**

| ***Cell line*** | ***IC***_{***10***}^{***1***} ***(µg*/*ml medium)*** | ***IC***_{***50***}^{***2***} ***(µg*/*ml medium)*** | ***IC***_{***90***}^{***3***} ***(µg*/*ml medium)*** | ***IC***_{***90***}***lIC***_{***10***}^{***4***} |
|---|---|---|---|---|
| MCF7 | 0.6 | 1.7 | 10 | 16.7 |
| BV173 | 0.4 | 1.8 | 7 | 17.5 |
| CC531 | 0.8 | 3.3 | 12 | 15.0 |
| U87-MG | 1.0 | 9 | 100 | 100 |
| K562 | 5 | 11 | 180 | 36 |
| SKW-3 | 3.1 | 20 | 700 | 226 |
| HEp2 | 5 | 21 | 100 | 20 |
| NCI-H460 | 4 | 21 | 150 | 38 |
| PC3 | 3.5 | 26 | >1000 | >300 |
| MDA-MB231 | 5 | 33 | 100 | 20 |
| HT29 | 8 | 40 | 350 | 44 |
| U333 | 7 | 65 | 300 | 43 |
| SAOS2 | 20 | 66 | 1000 | 50 |
| LAMA84 | 10 | 90 | 600 | 60 |
| HL60 | 30 | 90 | 1000 | 33 |
| CML-T1 | 2.5 | 160 | 1000 | 400 |
| AR230 | 17 | 170 | 700 | 41 |

| | | | | |
|---|---|---|---|---|
| ¹inhibitory concentration 10 (concentration inhibiting the cell growth by 10%), as assessed by MTT-assay (Konstantinov et al., Int.J.Cancer 77 (1998), pp. 778-786). | | | | |
| ²inhibitory concentration 50 (Concentration inhibiting the cell growth by 50%), as assessed by MTT-assay | | | | |
| ³inhibitory concentration 90 (Concentration inhibiting the cell growth by 90%), as assessed by MTT-assay | | | | |
| ⁴ratio of IC₉₀ and IC₁₀ values | | | | |

**Table 2: Comparison of the derived amino acid sequence of riproximin of US-origin with the published sequence of 7 other type II RIP proteins.**

| ***Type II RIP*** | ***Riproximin*** | |
|---|---|---|
| | ***% similarity***^{***1***} | ***% identity***^{***1***} |
| Ricin | 57.4 - 57.7 | 49.3 - 49.8 |
| RCA | 54.3 | 47.0 - 47.2 |
| Abrin | 49.1 - 49.7 | 42.9 - 43.5 |
| Viscumin | 52.9 - 53.2 | 45.2 - 45.3 |
| Ebulin | 48.7 - 48.9 | 40.9 - 41.1 |
| Nigrin1 | 46.2 - 46.4 | 38.2 - 38.9 |
| Cinnamomin | 54.3 - 54.7 | 46.3 - 46.6 |

| | | |
|---|---|---|
| ¹The percent similarity and identity depend on the allelic status of riproximin and are both given as range. | | |

## Claims

1. A nucleic acid molecule encoding the plant type II ribosome-inactivating protein (RIP) riproximin exhibiting anti-neoplastic activity or a polypeptide exhibiting a biological property of riproximin, which is:
(a) a nucleic acid molecule encoding a polypeptide that comprises the amino acid sequence as depicted in Figure 7 (SEQ ID NOs:3 or 4);
(b) a nucleic acid molecule comprising the nucleotide sequence as depicted in Figure 6(a)(SEQ ID NO:1) or (b)(SEQ ID NO:2);
(c) a nucleic acid molecule encoding a polypeptide the sequence of which shows at least 70% identity to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code; or
(e) a nucleic acid molecule, which represents a fragment or a variant of a nucleic acid molecule of (a) to (d).

2. A recombinant vector containing the nucleic acid molecule of claim 1

3. The recombinant vector of claim 2 wherein the nucleic acid molecule is operatively linked to regulatory elements allowing transcription and synthesis of a translatable RNA in prokaryotic and/or eukaryotic host cells.

4. A recombinant host cell which contains the recombinant vector of claim 2 or 3.

5. The recombinant host cell of claim 4, which is a bacterial cell or an eukaryotic cell, wherein the eukaryotic cell is, for example, a mammalian cell, an insect cell, a yeast cell or a plant cell.

6. A polypeptide exhibiting a biological property of riproximin which comprises an amino acid sequence which shows at least 70% identity to one of the amino acid sequences as depicted in Fig. 7 (SEQ ID NOs:3 or 4).

7. The polypeptide according to claim 6 which is encoded by a nucleic acid molecule of claim 1.

8. A method of producing a polypeptide exhibiting a biological property of riproximin comprising:
(a) culturing the recombinant host cell of claim 4 or 5 under conditions such that said polypeptide is expressed; and
(b) recovering said polypeptide.

9. A polypeptide produced by the method of claim 8.

10. A conjugate comprising the polypeptide of anyone of the claims 6, 7 or 9.

11. The conjugate of claim 10 which is an immunoconjugate.

12. An antibody that binds specifically to the polypeptide of any one of the claims 6, 7 or 9.

13. The nucleic acid molecule of claim 1, the polypeptide of claim 6, 7 or 9, the conjugate of claim 10 or 11, or the antibody of claim 12 which is detectably labeled.

14. The nucleic acid molecule, the polypeptide, the conjugate, or the antibody of claim 13, wherein the label is a radioisotope, a bioluminescent compound, a chemiluminescent compound, a fluorescent compound, a metal chelate, or an enzyme.

15. A pharmaceutical composition comprising a nucleic acid molecule of claim 1, a polypeptide of claim 6, 7 or 9, a conjugate of claim 10 or 11, or a recombinant vector of any one of claims 2 to 4 and a pharmaceutically acceptable excipient, diluent or carrier.

16. Use of a nucleic acid molecule of claim 1, a polypeptide of claim 6, 7 or 9 or a plant extract containing the polypeptide of claim 6, 7 or 9, a conjugate of claim 10 or 11, or a recombinant vector of any one of claims 2 to 4 for the preparation of a pharmaceutical composition for treatment of a tumor or for immunomodulation.

17. A plant extract comprising a polypeptide exhibiting a biological property of riproximin, wherein said polypeptide comprises an amino acid sequence which shows at least 70% identity to one of the amino acid sequences as depicted in Fig. 7 (SEQ ID NOs:3 or 4).


### SEQUENCE LISTING

<110> Deutsches Krebsforschungszentrum stiftung des öffentlichen Rechts
<120> Riproximin, a novel type II ribosome-inactivating protein and uses thereof
<130> K 3205EP
<160> 4
<170> PatentIn version 3.2
<210> 1
   <211> 1878
   <212> DNA
   <213> ximenia americana
<400> 1
   cgcctggttg tggtggagaa ctggcttgrt cggaggacct gtttggcttg gcsagctaat      60
   caacatgccg actactacca aaccggtact ggaagaccca gttgtggtcg tcgatcatca     120
   ccaaaccacg ackcctrcag aaccaccaat tagtaccgat gcggttctaa tccacagatc     180
   tggacgaggg gamgaagaaa aagacagcat atccagtaca gtcctgggca tcgactatcc     240
   cacattcaga ttgagcctca gcggcctcac ccaaagagac tacaaccagt tcgtgagaga     300
   tctccgggat agattggcaa ggcacaatga ccggcggcac ggagtgccgg ttctgcgtca     360
   acccggcrcg gtgctcgaaa acgagcggtt tattttagtg gaactmacga acatggcaga     420
   ggatcrgacc atgacgtggg ccatagacgt caccaacctc tacttagtgg gttatcaagt     480
   cggaraccgt tcctacttct ttcgaggtgy cccggaggaa gcaacccgca atctcttcam     540
   cgacacaacg csccartcct acgatttcac cggcgactac acgcaactcc gacckcgcgc     600
   aggagttgga aayatagaca gtattaccct gggattcaac cagctagacg aatccatcaa     660
   ttacctgttc cgtcactccg gccaaaycca tacggaagaa gaaaagaata agcaggccac     720
   cgccatgata gtttgcattg tgatgattgc ggaggcggtg cgcttccggt acatccaggg     780
   aacgctggcc ggaaccatta ttggagacgg acgatayaga agctttaaca acaacggtgc     840
   tgtggccagg ttcataaaca cgtggcagga cctctcccaa gccatccagg aatccaacga     900
   agatggggtc tttcctaacc aaatccgatt gcagtckccy acttatgctg agtattgggc     960
   gaccaacgtg atgcccaatt tagttgcggc cattgggatc atgwtatact tctgtgcaaa    1020
   gcccccyccg cctcwatctg tcgccagtac cgttggttat aacggccaaa rttgtgcyga    1080
   tccacagcca acggtccgca tcagcggtcg agatggaaag tgtgccgatg tgaagggcgg    1140
   atactacaac gatggagacc caataatctt gtggccgtgt aaatccaaca ccgatgcgaa    1200
   tcaactgtgg atcttgaaga aagacgggac tattcgatcc aagggaaagt gcttgagcac    1260
   ttcgtttggc cgataccatt tcatctccac cgtcatgatc tacgactgca gcatagcgac    1320
   aaaggccgcc acgcgttgga aagtgtggga taatggaacg atcataaatt ctgcatcctc    1380
   gcttgttttg actkcggagt tgggaacgag cgggacgccg ctcactgtga gggccaattc    1440
   gtactccacg agccaaagtt ggcttccttc caatttttcg gagccttttg tagtgagttc 1500
   yattctgggg ctcaaagatc tctgcctaca agcaactgga aatggggtgt ggctggagca 1560
   atgtgtgacg aacaaggcgg agcagaagtg ggctctcttt ccagatcgtt caatacggcc 1620
   ttattcagat cgagatcttt gcctctcttg taatactgat gaaacttccc atcaattgaa 1680
   catcgtcaat cttcttagct gtggttctgc gtcgcctggt caacactgga tgttcagaaa 1740
   taatggcgat gttttaaaca tatactctgg rttggttatg gatgtgaaag rttccaatcc 1800
   aagccttcac caaatacttg cgtggccgcc taacggaggt cgtaaccaaa aatggatggt 1860
   ggttgtctga catgcaca 1878
<210> 2
   <211> 1590
   <212> DNA
   <213> ximenia americana
<400> 2
   accaattcgt gagagatytc agggatagat tggcaaggca caatgaccgg cggcacggag      60
   tgccggttct gcgtcaaccc ggcacggtgc ccgaaaacga gcggtttatt ttagtggaac     120
   tcacgaacat ggcagaggat cggaccatga cgtgggccat agacgtcacc ratctctact     180
   tggtgggtta tcgagttggg gacttttcct acttctttcg aggtgccccg gaggaagcaa     240
   ccggcaatct cttcaccggt acaacgcccc aatcctacga tttcaccggc gactacacgc     300
   aactccgacc gcgcgcagga gttggaaaca tagacagtat taccctggga ttcaaccagc     360
   tagacgaatc catcaattac ctgttccgtc actccggcca aacccatacg gaagaagaaa     420
   agaataagca ggccaccgcc atgatagttt gcattgtgat gattgcggag gcggtgcgct     480
   tccggtacat ccagggaagg ctggccctaa ccattgaaga cggacgatat ggaagcttta     540
   acggcracgg tgctgtggcc aggttcataa acacgtggca ggrcctctct caagccatcc     600
   aggaatccaa cgaagatggg gtctttccta accaaatccg attgcagtcc ccaacttatg     660
   ctgagtattg ggtgaccaac gtgatgccca atttagttgc ggccattggg atcatgttat     720
   acttctgtgc aaaaccccca ccgcctcaat ctgtcgccag taccgttggt tataacggcc    780
   aaacttgtgc agatccacag ccaacrgtcc gcatcagcgg tcgagatgga aagtgtgccg     840
   atgttaaggg cggatactac aacgatggag acccaataat cttgtggccg tgtaaatcca     900
   acaccgatgc gaatcaactg tggaccttga agaaagacgg gactattcga tctaagggaa     960
   agtgcttgag cacttcgttt ggtcgatacc atttcatctc caccgtcatg atccacgact    1020
   gcagcatcgc gacaaaagct gccacgcgtt ggaaagtatg ggacaacgga acgatcataa    1080
   attccgcgtc gtcgctcgtt ttgactgcgg agttgggaac aagcgggacg ccgctcactg    1140
   tgagggccaa ttcgtactcc acgagccaaa gttggcttcc ttccaatggt tcggagcctt    1200
   ttgtagtgag ttctattccg gggctcaagg gtctctgctt acaagcaact ggaaatgggg    1260
   tgtggctgga gcaatgtgtg acgaacaagg cggagcagaa gtgggctctc tttccagrtc    1320
   gttcaatacg gccttattca gatcgagatc tttgcctctc ttgtaatact gatgaaactt    1380
   cccatcaatt gaacatcgtc aatcttctta gctgtggttc tgcgtcgcct ggtcaacact    1440
   ggatgttcag aaataatggc gatgttttaa acatatactc tggattggtt atggatgtga    1500
   aagattccaa tccaagcctt caccaaatac ttgcgtggcc gcctaacgga ggtcgtaacc    1560
   aaaaatggct ggtggttgtc tgacatgcac                                     1590
<210> 3
   <211> 601
   <212> PRT
   <213> ximenia americana
<220>
   <221> VARIANT
   <222> (25)..(25)
   <223> Ala or Thr
<220>
   <221> VARIANT
   <222> (43)..(43)
   <223> Glu or Asp
<220>
   <221> VARIANT
   <222> (102)..(102)
   <223> Ala or Thr
<220>
   <221> VARIANT
   <222> (121)..(121)
   <223> Arg or Gln
<220>
   <221> VARIANT
   <222> (141)..(141)
   <223> Asp or Asn
<220>
   <221> VARIANT
   <222> (159)..(159)
   <223> Asn or Thr
<220>
   <221> VARIANT
   <222> (208)..(208)
   <223> Ile or Thr
<220>
   <221> VARIANT
   <222> (314)..(314)
   <223> Ile or Leu
<220>
   <221> VARIANT
   <222> (324)..(324)
   <223> Gln or Leu
<220>
   <221> VARIANT
   <222> (336)..(336)
   <223> Ser or Asn
<220>
   <221> VARIANT
   <222> (444)..(444)
   <223> Ala or ser
<400> 3
   Met Pro Thr Thr Thr Lys Pro Val Leu Glu Asp Pro Val Val Val Val
   1               5                   10                  15
   Asp His His Gln Thr Thr Thr Pro Ala Glu Pro Pro Ile Ser Thr Asp
               20                  25                  30
   Ala Val Leu Ile His Arg Ser Gly Arg Gly Glu Glu Glu Lys Asp Ser
           35                  40                  45
   Ile Ser Ser Thr Val Leu Gly Ile Asp Tyr Pro Thr Phe Arg Leu Ser
       50                  55                  60
   Leu Ser Gly Leu Thr Gln Arg Asp Tyr Asn Gln Phe Val Arg Asp Leu
   65                  70                  75                  80
   Arg Asp Arg Leu Ala Arg His Asn Asp Arg Arg His Gly Val Pro Val
                   85                  90                  95
   Leu Arg Gln Pro Gly Ala Val Leu Glu Asn Glu Arg Phe Ile Leu Val
               100                 105                 110
   Glu Leu Thr Asn Met Ala Glu Asp Arg Thr Met Thr Trp Ala Ile Asp
           115                 120                 125
   Val Thr Asn Leu Tyr Leu Val Gly Tyr Gln Val Gly Asp Arg Ser Tyr
       130                 135                 140
   Phe Phe Arg Gly Val Pro Glu Glu Ala Thr Arg Asn Leu Phe Asn Asp
   145                 150                 155                 160
   Thr Thr Arg Gln Ser Tyr Asp Phe Thr Gly Asp Tyr Thr Gln Leu Arg
                   165                 170                 175
   Pro Arg Ala Gly Val Gly Asn Ile Asp Ser Ile Thr Leu Gly Phe Asn
               180                 185                 190
   Gln Leu Asp Glu Ser Ile Asn Tyr Leu Phe Arg His Ser Gly Gln Ile
           195                 200                 205
   His Thr Glu Glu Glu Lys Asn Lys Gln Ala Thr Ala Met Ile Val Cys
       210                 215                 220
   Ile Val Met Ile Ala Glu Ala Val Arg Phe Arg Tyr Ile Gln Gly Thr
   225                 230                 235                 240
   Leu Ala Gly Thr Ile Ile Gly Asp Gly Arg Tyr Arg Ser Phe Asn Asn
                   245                 250                 255
   Asn Gly Ala Val Ala Arg Phe Ile Asn Thr Trp Gln Asp Leu Ser Gln
   260 265 270
   Ala Ile Gln Glu Ser Asn Glu Asp Gly Val Phe Pro Asn Gln Ile Arg
   275 280 285
   Leu Gln Ser Pro Thr Tyr Ala Glu Tyr Trp Ala Thr Asn Val Met Pro
   290 295 300
   Asn Leu Val Ala Ala Ile Gly Ile Met Ile Tyr Phe Cys Ala Lys Pro
   305 310 315 320
   Pro Pro Pro Gln Ser Val Ala Ser Thr Val Gly Tyr Asn Gly Gln Ser
   325 330 335
   Cys Ala Asp Pro Gln Pro Thr Val Arg Ile Ser Gly Arg Asp Gly Lys
   340 345 350
   Cys Ala Asp Val Lys Gly Gly Tyr Tyr Asn Asp Gly Asp Pro Ile Ile
   355 360 365
   Leu Trp Pro Cys Lys Ser Asn Thr Asp Ala Asn Gln Leu Trp Ile Leu
   370 375 380
   Lys Lys Asp Gly Thr Ile Arg Ser Lys Gly Lys Cys Leu Ser Thr Ser
   385 390 395 400
   Phe Gly Arg Tyr His Phe Ile Ser Thr Val Met Ile Tyr Asp Cys Ser
   405 410 415
   Ile Ala Thr Lys Ala Ala Thr Arg Trp Lys Val Trp Asp Asn Gly Thr
   420 425 430
   Ile Ile Asn Ser Ala Ser Ser Leu Val Leu Thr Ala Glu Leu Gly Thr
   435 440 445
   Ser Gly Thr Pro Leu Thr Val Arg Ala Asn Ser Tyr Ser Thr Ser Gln
   450 455 460
   Ser Trp Leu Pro Ser Asn Phe Ser Glu Pro Phe Val Val Ser Ser Ile
   465 470 475 480
   Leu Gly Leu Lys Asp Leu Cys Leu Gln Ala Thr Gly Asn Gly Val Trp
   485 490 495
   Leu Glu Gln Cys Val Thr Asn Lys Ala Glu Gln Lys Trp Ala Leu Phe
   500 505 510
   Pro Asp Arg Ser Ile Arg Pro Tyr Ser Asp Arg Asp Leu Cys Leu Ser
   515 520 525
   Cys Asn Thr Asp Glu Thr Ser His Gln Leu Asn Ile Val Asn Leu Leu
   530 535 540
   Ser Cys Gly Ser Ala Ser Pro Gly Gln His Trp Met Phe Arg Asn Asn
   545 550 555 560
   Gly Asp Val Leu Asn Ile Tyr Ser Gly Leu Val Met Asp Val Lys Gly
   565 570 575
   Ser Asn Pro Ser Leu His Gln Ile Leu Ala Trp Pro Pro Asn Gly Gly
   580 585 590
   Arg Asn Gln Lys Trp Met Val Val Val
   595 600
<210> 4
   <211> 526
   <212> PRT
   <213> ximenia americana
<220>
   <221> VARIANT
   <222> (6)..(6)
   <223> Leu or Phe
<220>
   <221> VARIANT
   <222> (57)..(57)
   <223> Asn or Asp
<220>
   <221> VARIANT
   <222> (182)..(182)
   <223> Asn or Asp
<220>
   <221> VARIANT
   <222> (194)..(194)
   <223> Asp or Gly
<220>
   <221> VARIANT
   <222> (439)..(439)
   <223> Asp or Gly
<400> 4
   Gln Phe Val Arg Asp Leu Arg Asp Arg Leu Ala Arg His Asn Asp Arg
   1               5                   10                  15
   Arg His Gly Val Pro Val Leu Arg Gln Pro Gly Thr Val Pro Glu Asn
               20                  25                  30
   Glu Arg Phe Ile Leu Val Glu Leu Thr Asn Met Ala Glu Asp Arg Thr
           35                  40                  45
   Met Thr Trp Ala Ile Asp Val Thr Asn Leu Tyr Leu Val Gly Tyr Arg
       50                  55                  60
   Val Gly Asp Phe Ser Tyr Phe Phe Arg Gly Ala Pro Glu Glu Ala Thr
   65 70 75 80
   Gly Asn Leu Phe Thr Gly Thr Thr Pro Gln Ser Tyr Asp Phe Thr Gly
   85 90 95
   Asp Tyr Thr Gln Leu Arg Pro Arg Ala Gly Val Gly Asn Ile Asp Ser
   100 105 110
   Ile Thr Leu Gly Phe Asn Gln Leu Asp Glu Ser Ile Asn Tyr Leu Phe
   115 120 125
   Arg His Ser Gly Gln Thr His Thr Glu Glu Glu Lys Asn Lys Gln Ala
   130 135 140
   Thr Ala Met Ile Val Cys Ile Val Met Ile Ala Glu Ala Val Arg Phe
   145 150 155 160
   Arg Tyr Ile Gln Gly Arg Leu Ala Leu Thr Ile Glu Asp Gly Arg Tyr
   165 170 175
   Gly Ser Phe Asn Gly Asn Gly Ala Val Ala Arg Phe Ile Asn Thr Trp
   180 185 190
   Gln Asp Leu Ser Gln Ala Ile Gln Glu Ser Asn Glu Asp Gly Val Phe
   195 200 205
   Pro Asn Gln Ile Arg Leu Gln Ser Pro Thr Tyr Ala Glu Tyr Trp Val
   210 215 220
   Thr Asn Val Met Pro Asn Leu Val Ala Ala Ile Gly Ile Met Leu Tyr
   225 230 235 240
   Phe Cys Ala Lys Pro Pro Pro Pro Gln Ser Val Ala Ser Thr Val Gly
   245 250 255
   Tyr Asn Gly Gln Thr Cys Ala Asp Pro Gln Pro Thr Val Arg Ile Ser
   260 265 270
   Gly Arg Asp Gly Lys Cys Ala Asp Val Lys Gly Gly Tyr Tyr Asn Asp
   275 280 285
   Gly Asp Pro Ile Ile Leu Trp Pro Cys Lys Ser Asn Thr Asp Ala Asn
   290 295 300
   Gln Leu Trp Thr Leu Lys Lys Asp Gly Thr Ile Arg Ser Lys Gly Lys
   305 310 315 320
   Cys Leu Ser Thr Ser Phe Gly Arg Tyr His Phe Ile Ser Thr Val Met
   325 330 335
   Ile His Asp Cys Ser Ile Ala Thr Lys Ala Ala Thr Arg Trp Lys Val
               340                 345                 350
   Trp Asp Asn Gly Thr Ile Ile Asn Ser Ala Ser Ser Leu Val Leu Thr
           355                 360                 365
   Ala Glu Leu Gly Thr Ser Gly Thr Pro Leu Thr Val Arg Ala Asn Ser
       370                 375                 380
   Tyr Ser Thr Ser Gln Ser Trp Leu Pro Ser Asn Gly Ser Glu Pro Phe
   385                 390                 395                 400
   Val Val Ser Ser Ile Pro Gly Leu Lys Gly Leu Cys Leu Gln Ala Thr
                   405                 410                 415
   Gly Asn Gly Val Trp Leu Glu Gln Cys Val Thr Asn Lys Ala Glu Gln
               420                 425                 430
   Lys Trp Ala Leu Phe Pro Asp Arg Ser Ile Arg Pro Tyr Ser Asp Arg
           435                 440                 445
   Asp Leu Cys Leu Ser Cys Asn Thr Asp Glu Thr Ser His Gln Leu Asn
       450                 455                 460
   Ile Val Asn Leu Leu Ser Cys Gly Ser Ala Ser Pro Gly Gln His Trp
   465                 470                 475                 480
   Met Phe Arg Asn Asn Gly Asp Val Leu Asn Ile Tyr Ser Gly Leu Val
                   485                 490                 495
   Met Asp Val Lys Asp Ser Asn Pro Ser Leu His Gln Ile Leu Ala Trp
               500                 505                 510
   Pro Pro Asn Gly Gly Arg Asn Gln Lys Trp Leu Val Val Val
           515                 520                 525